(19) Europäisches Patentamt
European Patent Office
Office européen des brevets

(11) EP 2 251 024 A1

(12) **EUROPEAN PATENT APPLICATION**
published in accordance with Art. 153(4) EPC

(43) Date of publication:
**17.11.2010 Bulletin 2010/46**

(21) Application number: **09703148.8**

(22) Date of filing: **21.01.2009**

(51) Int Cl.:
***A61K 36/28*** *(2006.01)* ***A23L 1/30*** *(2006.01)*
***A61K 8/97*** *(2006.01)* ***A61K 36/00*** *(2006.01)*
***A61P 19/06*** *(2006.01)* ***A61P 43/00*** *(2006.01)*
***C12N 9/99*** *(2006.01)*

(86) International application number:
**PCT/JP2009/050797**

(87) International publication number:
**WO 2009/093584 (30.07.2009 Gazette 2009/31)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **23.01.2008 JP 2008012910**
**15.02.2008 JP 2008034209**

(71) Applicant: **Kaneka Corporation**
**Kita-ku**
**Osaka-shi, Osaka 530-8288 (JP)**

(72) Inventors:
- **HONDA, Shinichi**
**Takasago-shi**
**Hyogo 676-8688 (JP)**
- **TANAKA, Hozumi**
**Takasago-shi**
**Hyogo 676-8688 (JP)**
- **KISHIDA, Hideyuki**
**Takasago-shi**
**Hyogo 676-8688 (JP)**
- **KITAGAWA, Masayasu**
**Takasago-shi**
**Hyogo 676-8688 (JP)**

(74) Representative: **Jaenichen, Hans-Rainer**
**Vossius & Partner**
**Siebertstrasse 4**
**81675 München (DE)**

(54) **PLANT-ORIGIN DRUG FOR PREVENTING OR IMPROVING HYPERURICEMIA**

(57) This invention provides an agent for preventing or ameliorating hyperuricemia comprising, as an active ingredient, an extract of at least one type of plant raw material selected from the group consisting of *Artemisia*, *Saussurea involucrate*, chrysanthemum, guava, cudweed *(Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative or a glycoside thereof.

EP 2 251 024 A1

## Description

### Technical Field

The present invention relates to an agent for preventing or ameliorating hyperuricemia and a xanthine oxidase inhibitory agent comprising, as an active ingredient, an ingredient derived from at least one type of plant raw material selected from the group consisting of *Artemisia*, *Saussurea involucrate*, chrysanthemum, guava, cudweed *(Gnaphalium affine*), blue mallow, oregano, *Glechoma hederacea*, mint, *Millettia reticulata*, peanuts, and Pycnogenol or a chalcone derivative.

### Background Art

In recent years, the number of people having hyperuricemia or symptoms of gout caused by hyperuricemia has been increasing, due to a rapid increase in intake of high-calorie, high-protein, and high-fat foods as well as heightened stress. At present, it is said that approximately 20% of adult males have hyperuricemia.

In general, a condition in which the uric acid level in the blood is 7.0 mg/dL or higher is referred to as hyperuricemia. Usually, a person does not experience subjective symptoms merely while under the condition of hyperuricemia. If such condition is left untreated for a long period of time, however, urate in the blood is crystallized, and a person experiences attacks of gout, involving acute pain upon urate accumulation in joints or the like. As the symptom further advances, it is known that a gouty tophus may arise subcutaneously and urinary calculus may easily develop due to accumulated uric acid crystals in the kidney or urinary tract. If such condition is prolonged, kidney diseases such as kidney failures occur. Thus, adequate regulation of the uric acid level in the blood is a fundamental aspect of prevention and amelioration of hyperuricemia, including gout. Reduction of the uric acid level in the blood to an adequate level is considered to be effective for a patient with severe gout in reducing attacks of gout, inhibiting chronicity of gout, and preventing and ameliorating kidney diseases (Non-Patent Document 1).

The amount of uric acid in the body is generally maintained at an adequate level because of the balance between the excretion and production thereof. If such balance is disrupted due to factors such as disorderly daily habits or stress, however, the uric acid level is elevated. In order to maintain the uric acid level at an adequate level, accordingly, inhibition of uric acid production is considered to be important. Uric acid in the body is produced from xanthine by the action of xanthine oxidase (Non-Patent Document 2). Thus, it is expected that inhibition of the action of xanthine oxidase leads to inhibition of uric acid production in the body, reduction of the uric acid level in the blood, and prevention or amelioration of gout or hyperuricemia.

To date, probenecid (a uricosuric agent), allopurinol (an inhibitor of uric acid production), and the like have been used in order to regulate the uric acid level in the blood, although such agents have been disadvantageous in terms of transiency of effects or side effects imposed upon the kidney. Therefore, development of xanthine oxidase inhibitors that have excellent effects of inhibiting xanthine oxidase and reducing the uric acid level in the blood, are highly safe, and can be administered for a long period of time has been awaited.

Many compounds that have xanthine oxidase inhibitory activity have heretofore been reported; however, many such compounds were chemically synthesized and thus cannot be regarded as being satisfactorily safe. Although there are reports regarding research and development of xanthine oxidase inhibitors derived from nature (Patent Documents 1 and 2), the effects thereof for inhibiting xanthine oxidase were insufficient.

Patent Document 1: JP Patent Publication (kokai) No. 2006-36787 A
Patent Document 2: JP Patent Publication (kokai) No. 2007-45784 A
Non-patent Document 1: Mebio, 2000, vol. 17, pp. 24 to 29
Non-patent Document 2: Purine-pyrimidine taisha (metabolism), 1994, vol. 18, pp. 1 to 10

### Disclosure of the Invention

### Problem to be Solved by the Invention

Under the above circumstances, the present invention is intended to provide an agent for preventing or ameliorating hyperuricemia and a xanthine oxidase inhibitory agent, both of which are highly safe.

### Means for Solving the Problem

The present inventors have conducted concentrated studies in order to solve the above problem. As a result, they discovered that extracts of *Artemisia, Saussurea involucrate*, chrysanthemum, guava, cudweed (*Gnaphalium af-*

*fine*), blue mallow, oregano, *Glechoma hederacea*, mint, *Millettia reticulata*, and peanuts and Pycnogenol have xanthine oxidase inhibitory activity and such substances are very effective for the prevention and amelioration of hyperuricemia, thereby completing the present invention. Further, they also discovered that chalcone derivatives, and okanin or okanin glycoside in particular, had xanthine oxidase inhibitory activity and were very useful as agents for preventing or ameliorating hyperuricemia. This has led to the completion of the present invention.

Specifically, the present invention is as follows.

[1] An agent for preventing or ameliorating hyperuricemia comprising, as an active ingredient, an extract of at least one type of plant raw material selected from the group consisting of *Artemisia*, *Saussurea involucrate*, chrysanthemum, guava, cudweed (*Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative represented by general formula (1):

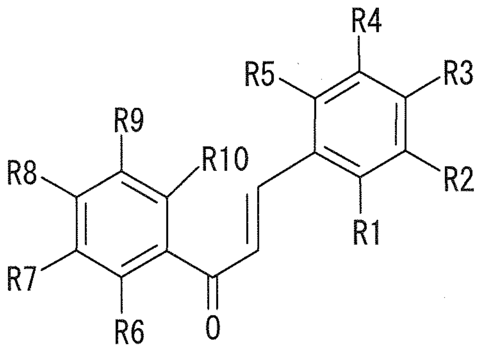

(1)

wherein R1 to R10 are each independently H, OH, or $OCH_3$, or a glycoside thereof.

[2] A xanthine oxidase inhibitory agent comprising, as an active ingredient, an extract of at least one type of plant raw material selected from the group consisting of *Artemisia*, *Saussurea involucrate*, chrysanthemum, guava, cudweed *(Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative represented by general formula (1):

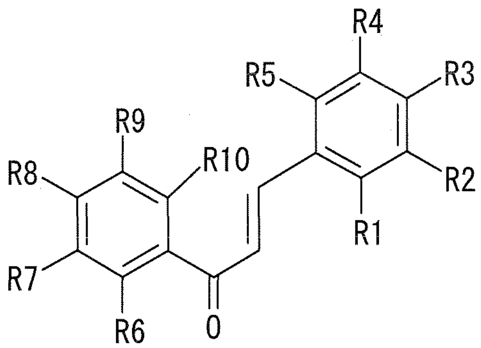

(1)

wherein R1 to R10 are each independently H, OH, or $OCH_3$, or a glycoside thereof.

[3] The agent according to [1] or [2], wherein the organic solvent is ethanol.

[4] The agent according to [1] or [2], wherein the chalcone derivative represented by general formula (1) or the glycoside thereof is derived from a plant of the genus *Bidens*.

[5] The agent according to [1] or [2], wherein the chalcone derivative represented by general formula (1) or the glycoside thereof is okanin or an okanin glycoside.

[6] A food or beverage product comprising the agent according to any of [1] to [5].

[7] The food or beverage product according to [6], which is a functional food.
[8] The food or beverage product according to [6], which is a food for specified health use.
[9] A cosmetic or pharmaceutical product comprising the agent according to any of [1] to [5].

## Effects of the Invention

The agent for preventing or ameliorating hyperuricemia and the xanthine oxidase inhibitory agent according to the present invention are derived from a plant raw material that people are familiar with eating. Thus, such agents are highly safe and effective for prevention or amelioration of hyperuricemia because of excellent xanthine oxidase inhibitory activity.

## Best Modes for Carrying out the Invention

Hereafter, the present invention is described in detail.
The agent for preventing or ameliorating hyperuricemia according to the present invention comprises, as an active ingredient, an extract of at least one type of plant raw material selected from the group consisting of *Artemisia, Saussurea involucrate*, chrysanthemum, guava, cudweed (*Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative or a glycoside thereof. Such agent can be used for prevention or amelioration of hyperuricemia. Further, such agents can be used to regulate the uric acid level in the blood so as to achieve an adequate level thereof.
In the present invention, the term "prevention of hyperuricemia" refers to prevention or slowing down (or reduction of a risk) of the condition of hyperuricemia (i.e., a uric acid level in the blood of 7.0 mg/dL or higher) as defined in the guidelines for treatment of hyperuricemia and gout of the Japanese Society of Gout and Nucleic Acid Metabolism (published in September, 2002). The term "amelioration of hyperuricemia" used herein refers to reduction of the uric acid level in the blood from that of the condition of hyperuricemia described above to the normal level or a level close thereto. The agent for preventing or ameliorating hyperuricemia of the present invention can be used for prevention or amelioration of gout symptoms via regulation of the uric acid level in the blood.
In the present invention, a composition comprising, as an active ingredient, an extract of at least one type of plant raw material selected from the group consisting of *Artemisia, Saussurea involucrate*, chrysanthemum, guava, cudweed *(Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative or a glycoside thereof can also be used as a xanthine oxidase inhibitory agent (xanthine oxidase inhibitor).
The xanthine oxidase inhibitory agent of the present invention inhibits the action of xanthine oxidase, thereby inhibiting uric acid production. Also, the xanthine oxidase inhibitory agent of the present invention can inhibit active oxygen generated when xanthine oxidase produces uric acid from xanthine. Further, the xanthine oxidase inhibitory agent of the present invention can be used for a variety of expected applications resulting from inhibition or suppression of the action of xanthine oxidase.
In the present invention, the effects of xanthine oxidase inhibition can be evaluated by a method in which a test substance (i.e., a candidate substance of a xanthine oxidase inhibitory agent) is administered to a mammalian animal or a mammalian animal is allowed to ingest the same and the effects thereof are assayed or a method in which a test substance is allowed to react with commercially available or prepared xanthine oxidase and inhibitory activity is assayed. In general, the method involving the use of commercially available or prepared xanthine oxidase is employed because of excellent sensitivity, reproducibility, and convenience.
Specifically, xanthine oxidase inhibitory activity can be assayed by, for example, using xanthine as a substrate and buttermilk-derived xanthine oxidase as an enzyme and detecting generated uric acid (Biol. Pharm. Bull., 2004, vol. 27, pp. 1414 to 1421). In the present invention, a sample exhibiting xanthine oxidase inhibitory activity higher than that of the solvent control by at least 40% in such assay system is evaluated as "having xanthine oxidase inhibitory activity."
Active ingredients or plant raw materials from which such active ingredients originate of the agent for preventing or ameliorating hyperuricemia and the xanthine oxidase inhibitory agent of the present invention (hereafter, such agents are collectively referred to as "the agent(s) of the present invention") are *Artemisia, Saussurea involucrate*, chrysanthemum, guava, cudweed (*Gnaphalium affine*), blue mallow, oregano, *Glechoma hederacea*, mint, *Millettia reticulata*, peanuts, Pycnogenol, and/or a chalcone derivative or a glycoside thereof. Such materials can be used alone or in combinations of two or more. Also, plant raw materials that are generally used for food may be used.
When *Artemisia* is used for the agent of the present invention, *Gaiyo* (leaves of *Artemisia princeps PAMP*), which is a crude drug originating from *Artemisia*, may also be used. When cudweed (*Gnaphalium affine*) is used for the agent of the present invention, *Gnaphalium affine* D. Don, which is a crude drug originating from cudweed *(Gnaphalium affine*), may also be used. When *Glechoma hederacea* is used for the agent of the present invention, *Desmodium*

*styracifolium* or *Glechoma hederacea L var. grandis*, which is a crude drug originating from *Glechoma hederacea*, may also be used. When *Millettia reticulata* is used for the agent of the present invention, *Millettia reticulata* originating from *Millettia reticulata* may also be used.

Entire parts, including terrestrial parts (e.g., leaves, stems, buds, flowers, xylem, and bark), subterranean parts (e.g., roots and tubers), seeds, resins, and other parts of plant raw materials, such as *Artemisia*, *Saussurea involucrate*, chrysanthemum, guava, cudweed *(Gnaphalium affine*), blue mallow, oregano, *Glechoma hederacea*, mint, *Millettia reticulata*, and peanuts, which are used for the agents of the present invention, can be used. In particular, use of the whole *Artemisia* plant, the whole *Saussurea involucrate* plant, chrysanthemum flowers, guava leaves, the whole cudweed *(Gnaphalium affine*) plant, blue mallow flowers, oregano leaves and flowers, the whole *Glechoma hederacea* plant, mint leaves, *Millettia reticulata* stems, and the astringent skin of peanuts is preferable.

In the present invention, an extract of *Artemisia, Saussurea involucrate*, chrysanthemum, guava, cudweed *(Gnaphalium affine*), blue mallow, oregano, *Glechoma hederacea*, mint, *Millettia reticulata*, or peanuts obtained with the use of an organic solvent or aqueous organic solvent can be used as an active ingredient of the agent of the present invention. In the present invention, methods for obtaining an extract of such plant raw material are not particularly limited. The extract can be obtained by, for example, soaking the plant raw material in an organic solvent or aqueous organic solvent, agitating or allowing the resultant to stand under ordinary pressure, and performing static separation, filtration, or centrifugation.

A solvent used for extraction in the present invention is an organic solvent or an aqueous organic solvent (i.e., a mixed solvent of an organic solvent and water). Such organic solvent is not particularly limited, and examples thereof include ethanol, ethyl acetate, acetone, hexane, and methanol. When such organic solvent is mixed with water, use of a water-soluble organic solvent is preferable.

From the viewpoint of the safe application of the resulting extract to food or pharmaceutical products, selection of ethanol or an aqueous ethanol solution as an organic solvent used for extraction is particularly preferable. When an aqueous ethanol solution is used as an extraction solvent in the present invention, the ethanol concentration is not particularly limited. For example, the ethanol concentration is 50% by weight or more, preferably 90% by weight or more, and more preferably 95% by weight or more of the solution.

Extraction can be carried out at generally -20°C to 100°C, usually 1°C to 90°C, and preferably 10°C to 70°C. The duration of extraction is usually 0.1 hours to 1 month, and preferably 0.5 hours to 7 days. The amount of the extraction solvent used is preferably 1 to 50 times greater, and more preferably 3 to 20 times greater than each 1 weight part of *Artemisia*, *Saussurea involucrate*, chrysanthemum, guava, cudweed (*Gnaphalium affine*), blue mallow, oregano, *Glechoma hederacea*, mint, *Millettia reticulata*, or peanuts. When the amount of the solvent is less than 1 time relative to the amount of the above plant raw material, the plant raw material cannot be sufficiently soaked in the solvent, and low recovery of the extract occurs. When the amount of the solvent is 50 times or more greater than the amount of the above plant raw material, the size of a vessel such as an extraction tank is increased, the energy required for solvent removal becomes very high, and the production cost is likely to increase.

In the present invention, an extract of *Artemisia, Saussurea involucrate*, chrysanthemum, guava, cudweed *(Gnaphalium affine*), blue mallow, oregano, *Glechoma hederacea*, mint, *Millettia reticulata*, or peanuts may be used in the form of an extracted solution, and the extract from which the solvent has been removed may be used. Further, the extract may be dissolved or suspended in another adequate solvent. Further, the extract of *Artemisia*, *Saussurea involucrate*, chrysanthemum, guava, cudweed *(Gnaphalium affine*), blue mallow, oregano, *Glechoma hederacea,* mint, *Millettia reticulata*, or peanuts can be used in the form of an extract, a crude extract, or a semi-purified extract in the present invention, provided that such extract does not contain impurities that are inappropriate for food or beverage products or for pharmaceutical products.

The agent of the present invention can comprise Pycnogenol as an active ingredient. Pycnogenol is an extract obtained from the bark of the French maritime pine (*Pinus pinaster* ssp. *atlantica*). "French maritime pine bark extract" or "Pycnogenol" that is generally commercially available may be used. Alternatively, an extract obtained from the bark of the French maritime pine in accordance with a conventional technique may be used. Also, supplements or health foods containing a large quantity of Pycnogenol can be used.

Further, the agent of the present invention can comprise a chalcone derivative or a glycoside thereof as an active ingredient. In general, the term "chalcone derivative" is a generic term for a compound having a chalcone skeleton (i.e., a β-phenol acrylophenon skeleton) represented by formula (2).

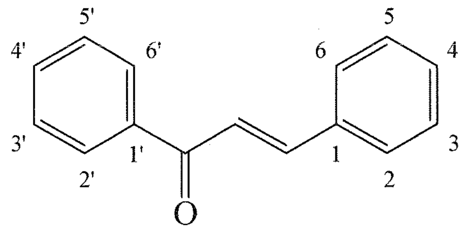

(2)

In the present invention, a chalcone derivative or a glycoside thereof in which at least one of position numbers 2 to 6 and 2' to 6' in formula (2) has been substituted with a hydroxyl or methoxy group is used as an active ingredient. Specifically, the chalcone derivative as an active ingredient of the agent of the present invention is a chalcone derivative or a glycoside thereof represented by general formula (1) in which R1 to R10 are each independently H, OH, or $OCH_3$. Hereafter, the chalcone derivative used in the present invention is described in greater detail.

In the present invention, substituents R1 to R10 in general formula (1) are not particularly limited, provided that such substituents each independently or in an arbitrary combination represent any of H, OH, or $OCH_3$. It should be noted that all of R1 to R10 do not simultaneously represent H, and at least one thereof should be substituted with OH or $OCH_3$.

In the present invention, also, at least one of R1 to R10 of the chalcone derivative is preferably OH, and it is more preferable that two or more thereof be OH. Also, at least three of R1 to R10 are preferably OH or $OCH_3$.

Specific examples of such chalcone derivative include, but are not limited to, butein (in general formula (1), R3, R4, R8, and R10 are each OH, and other residues are each H), lanceoletin (in general formula (1), R3, R4, R6, and R8 are each OH, R7 is $OCH_3$, and other residues are each H), okanin (in general formula (1), R3, R4, and R8 to R10 are each OH and other residues are each H), pedicellin (in general formula (1), R6 to R10 are each $OCH_3$ and other residues are each H), pedicin (in general formula (1), R6 and R9 are each OH, R7, R8, and R10 are each $OCH_3$, and other residues are each H), stillopsidin (in general formula (1), R3, R4, R6, R8, and R9 are each OH and other residues are each H), echinatin (in general formula (1), R3 and R8 are each OH, R5 is $OCH_3$, and other residues are each H), isoliquiritigenin (in general formula (1), R3, R8, and R10 are each OH and other residues are each H), and licochalcone B (in general formula (1), R3, R4, and R8 are each OH, R5 is $OCH_3$, and other residues are each H).

When a glycoside of the chalcone derivative is used in the present invention, a sugar residue derived from a monosaccharide or oligosaccharide comprising two or three sugars is selected. Representative examples of monosaccharides from which such sugar residues are derived include aldoses, such as glucose, mannose, arabinose, and xylose, ketoses, such as ribulose and xylulose, and deoxysugars, such as rhamnose and fucose. An oligosaccharide comprising two or more of the same or different monosaccharides linked by glycosidic bonds can be used. In the present invention, any such glycosides can be preferably used.

In the present invention, the most preferable agent comprising, as an active ingredient, the above-described chalcone derivative is an agent at least comprising okanin or an okanin glycoside.

The chalcone derivative used as an active ingredient of the agent of the present invention can be chemically synthesized. However, such chalcone derivative is preferably extracted from a natural substance such as a plant, from the viewpoint of the safety of human bodies. In such a case, chrysanthemum flowers or a plant of the genus *Bidens* is preferable as a plant raw material used for extraction. When a plant of the genus *Bidens* is used, use of *Bidens Pilosa* or *Bidens frondosa* is more preferable.

The form of the use of chrysanthemum flowers or the plant of the genus *Bidens* in the present invention is not particularly limited. For example, chrysanthemum flowers or a plant of the genus *Bidens* can be used in the form of a fresh plant, a dried plant, or a ground product of such dried plant. Extraction methods are not particularly limited, provided that the chalcone derivative represented by general formula (1) is extracted. For example, chrysanthemum flowers or a plant of the genus *Bidens* is dried and ground, and extraction is performed with the use of an extraction solvent. The chalcone derivative is then separated and purified from the extract via chromatography.

A solvent used for extraction in the present invention is not particularly limited, and preferable examples thereof include an organic solvent, such as ethanol, methanol, ethyl acetate, acetone, or hexane, and a mixed solvent of such organic solvent and water. When such organic solvent is mixed with water, use of a water-soluble organic solvent is preferable. From the viewpoint of safe application of the resulting extract to food or pharmaceutical products, it is particularly preferable to select ethanol or an aqueous ethanol solution.

After the extraction, the extraction solution used for hydrolysis with the use of an acid or enzyme may be subjected to chromatography to separate and purify the chalcone derivative. The extract comprising the chalcone derivative may be used without modification, as long as the extract does not contain inadequate impurities.

The chalcone derivative or a glycoside thereof and plant extracts comprising the same inhibit uric acid production

in the body via expression of xanthine oxidase inhibitory activity. Thus, the chalcone derivative or a glycoside thereof and plant extracts comprising the same are useful as agents for preventing or ameliorating hyperuricemia or gout symptoms. Further, the chalcone derivative or a glycoside thereof and plant extracts comprising the same can be used for preventing a variety of diseases resulting from hyperuricemia or gout, and the chalcone derivative or a glycoside thereof and plant extracts comprising the same exhibit effects of preventing or ameliorating diseases generated by active oxygen, such as inflammation, arteriosclerosis, cancerogenesis, brain disorders, or metabolic syndrome.

The active ingredient of the agent of the present invention; i.e., the extract of *Artemisia*, *Saussurea involucrate*, chrysanthemum, guava, cudweed *(Gnaphalium affine*), blue mallow, oregano, *Glechoma hederacea*, mint, *Millettia reticulata*, or peanuts obtained with the use of an organic solvent or aqueous organic solvent, Pycnogenol, or chalcone derivative or a glycoside thereof, can be subjected to a procedure such as deodorization or purification, provided that xanthine oxidase inhibitory activity is not lost.

As described above, the active ingredient of the agent of the present invention is an extract of at least one type of plant raw material selected from the group consisting of *Artemisia, Saussurea involucrate*, chrysanthemum, guava, cudweed *(Gnaphalium affine*), blue mallow, oregano, *Glechoma hederacea*, mint, *Millettia reticulata*, and peanuts obtained with the use of an organic solvent or aqueous organic solvent, Pycnogenol, or chalcone derivative or a glycoside thereof (hereafter, it may be referred to as the "active ingredient"). The agent of the present invention may comprise such active ingredient alone, or such active ingredient may be mixed with an additive such as a known carrier or adjuvant and prepared to result in a dosage form that can be easily taken (e.g., capsules, tablets, or granules). For the purpose of nutrient enrichment, various vitamins, such as vitamin A, C, D, or E, may be added. The content of the active ingredient in the agent of the present invention is not particularly limited, and it is preferably 0.1 % to 100% by weight, and more preferably 10% to 90% by weight.

The agent of the present invention can be used as a food or beverage product, cosmetic product, or pharmaceutical product comprising such ingredient.

The food or beverage product comprising the agent of the present invention is not particularly limited. For example, the agent is mixed with a food or beverage material and used for any general food or beverage products, such as confectionaries, such as chewing gum, chocolate, candies, jelly, biscuits, or crackers; frozen desserts, such as ice cream or ices; beverages, such as tea, soft beverages, nutrition-supplement beverages, or beauty beverages; noodles, such as Japanese wheat noodles, Chinese noodles, spaghetti, or instant noodles; kneaded products, such as steamed fish paste (Kamaboko), tube-shaped fish paste cake (chikuwa), or steamed cakes of smashed fish and yam (hanpen); seasonings, such as dressing, mayonnaise, or sauce; oil and fat products, such as margarine, butter, or cooking oil; bread, ham, soup, retort food products, or frozen food products. In particular, a food or beverage product comprising the chalcone derivative or a glycoside thereof can be provided in the form of chrysanthemum flower juice or a condensed extract of *Bidens biternata Merr. et Sherff* having the effects of preventing or ameliorating hyperuricemia or having xanthine oxidase inhibitory activity, for example. Further, the agent of the present invention may be prepared in the form of a health-promoting food such as a food for specified health use or a food with nutrient function claims, health food products, or supplements.

When such food or beverage product is ingested, the amount thereof is preferably 0.01 to 1,000 mg/kg of body weight, and more preferably 0.1 to 300 mg/kg of body weight, per day per adult in terms of the amount of the active ingredient.

When the agent of the present invention is used in the form of a cosmetic or pharmaceutical product, further, the dosage form is not particularly limited. For example, the agent can be in a powder form obtained via spray drying or lyophilization. Also, the agent can be used in the form of, for example, capsules such as hard or soft capsules, tablets exemplified by chewable tablets, granules, liquid preparations, powders, injection preparations, suppositories, and adhesive skin patches. Pharmaceutical preparations can be prepared by adequately adding other pharmacologically acceptable materials, such as excipients, disintegrators, lubricants, binders, antioxidants, coloring agents, antiaggregating agents, absorption promoters, solubilizers, or stabilizers.

The amount of such preparation to be administered varies depending on symptoms, the route of administration, the age, and the body weight of a person who takes such preparation, as well as other conditions. A preparation comprising an extract of at least one type of plant raw material selected from the group consisting of *Artemisia, Saussurea involucrate*, chrysanthemum, guava, cudweed (*Gnaphalium affine*), blue mallow, oregano, *Glechoma hederacea*, mint, *Millettia reticulata*, and peanuts obtained with the use of an organic solvent or aqueous organic solvent or Pycnogenol is administered in amounts of preferably 0.01 to 1,000 mg/kg of body weight, and more preferably 0.1 to 100 mg/kg of body weight, per day per adult in terms of the amount of the active ingredient. Such preparation is preferably administered in a single dose or several separate doses. The amount of a preparation comprising the chalcone derivative or a glycoside thereof is generally 0.01 g to 20 g, and preferably 0.05 g to 5 g per day, per adult in terms of the amount of the chalcone derivative to be administered.

Also, the agent of the present invention can be used in combination with another pharmaceutical product, a composition used for treating hyperuricemia, or food having xanthine oxidase inhibitory activity.

Examples

Hereafter, the present invention is described in greater detail with reference to examples, although the present invention is not limited to these examples.

(Example 1)

*Artemisia* (whole plant), *Saussurea involucrate* (whole plant), cudweed *(Gnaphalium affine*) (whole plant), *Glechoma hederacea* (whole plant), mint (leaves), and *Millettia reticulata* (stems) (purchased from Shinwa Bussan Kaisha Ltd.); chrysanthemum (flowers) and oregano (leaves and flowers) (purchased from Kaneka Sun Spice Co., Ltd.); blue mallow (flowers) (purchased from K. Kobayashi & Co., Ltd.); and guava (leaves) and peanuts (astringent skins) (purchased from retail stores) (1,000 g each) were soaked in 5 liters of an aqueous solution of 99.5% ethanol by volume. Agitation and extraction were carried out at 45°C for 6 hours and residues were removed by filtration to obtain extracted solutions. Further, the extracted solutions were concentrated under reduced pressure to remove solvents, and extracts were obtained. Commercially available Pycnogenol was used without modification.

(Example 2)

In order to evaluate the xanthine oxidase inhibitory activity of samples, the extracts obtained in Example 1 and Pycnogenol were dissolved in DMSO to a concentration of 100 mg/ml. The solutions of samples in DMSO were dissolved in 75 mM phosphate buffer (pH 7.5) to prepare solutions containing samples at concentrations of 400 μg/ml.

A sample solution (50 μl) and an enzyme solution comprising buttermilk-derived xanthine oxidase dissolved at 0.2 units/ml in phosphate buffer (50 μl) were added to a 96-well plate and treated at 25°C for 15 minutes. Thereafter, 100 μl of 0.4 mM xanthine solution was added and the reaction was allowed to proceed at 25°C for an additional 15 minutes. In this case, the final concentration of the sample was 100 μg/ml. The reaction was terminated with the addition of 20 μl of 1N hydrochloric acid. The absorbance at 295 nm was assayed using a microplate reader. As a solvent control, 50 μl of a solution comprising DMSO dissolved at 0.4% in phosphate buffer was used instead of the sample solution. The amount of uric acid (i.e., the reaction value) generated by xanthine oxidase in the reaction solution comprising the solvent control or the sample was determined by subtracting the absorbance assayed in the absence of xanthine oxidase from the absorbance assayed in the presence thereof. The xanthine oxidase inhibitory activity was determined by the following formula: Xanthine oxidase inhibitory activity (%) = {1 - (reaction value of test substance (sample) /reaction value of solvent control)} x 100

The xanthine oxidase inhibitory activities of the extracts of *Artemisia*, *Saussurea involucrate*, chrysanthemum, guava, cudweed *(Gnaphalium affine*), blue mallow, oregano, *Glechoma hederacea*, mint, *Millettia reticulata*, and peanuts obtained in Example 1 and Pycnogenol are shown in Table 1.

Table 1

| | Xanthine oxidase inhibitory activity (%) |
|---|---|
| *Artemisia* extract | 84.2 |
| *Saussurea involucrate* extract | 61.7 |
| Chrysanthemum extract | 64.1 |
| Guava extract | 63.6 |
| Cudweed *(Gnaphalium affine*) extract | 48.7 |
| Blue mallow extract | 51.1 |
| Oregano extract | 52.7 |
| *Glechoma hederacea* extract | 43.2 |
| Mint extract | 41.3 |
| *Millettia reticulata* extract | 49.4 |
| Peanuts extract | 47.4 |
| Pycnogenol | 46.6 |

As is apparent from Table 1, the extracts of *Artemisia, Saussurea involucrate*, chrysanthemum, guava, cudweed *(Gnaphalium affine*), blue mallow, oregano, *Glechoma hederacea*, mint, *Millettia reticulata*, and peanuts obtained with the use of ethanol in Example 1 and Pycnogenol exhibited potent xanthine oxidase inhibitory activities.

(Example 3)

In order to evaluate the xanthine oxidase inhibitory activity of samples, okanin (derived from *Bidens frondosa*) and allopurinol (manufactured by Wako Pure Chemical Industries, Ltd.) were dissolved in DMSO to a concentration of 100 mM. The solutions thereof in DMSO were dissolved in 75 mM phosphate buffer (pH 7.5) to prepare solutions containing samples at concentrations of 400 μg/ml.

The sample solution (50 μl) and the enzyme solution comprising buttermilk-derived xanthine oxidase dissolved at 0.2 units/ml in phosphate buffer (50 μl) were added to a 96-well plate and treated at 25°C for 15 minutes. Thereafter, 100 μl of 0.4 mM xanthine solution was added and the reaction was allowed to proceed at 25°C for an additional 15 minutes. In this case, the final concentration of the sample was 100 μM. The reaction was terminated with the addition of 20 μl of 1N hydrochloric acid. The absorbance at 295 nm was assayed using a microplate reader. As a solvent control, 50 μl of a solution comprising DMSO dissolved at 0.4% in phosphate buffer was used instead of the sample solution. The amount of uric acid (i.e., the reaction value) generated by xanthine oxidase in the reaction solution comprising the solvent control or the sample was determined by subtracting the absorbance assayed in the absence of xanthine oxidase from the absorbance assayed in the presence thereof. The xanthine oxidase inhibitory activity was determined by the following formula: Xanthine oxidase inhibitory activity (%) = {1 - (reaction value of test substance (sample) /reaction value of solvent control)} x 100

Further, sample concentrations were varied and the xanthine oxidase inhibitory activities of the samples at various concentrations were determined in the same manner. The $IC_{50}$ value of okanin relative to xanthine oxidase was compared with that of allopurinol used as an agent for treating hyperuricemia. The $IC_{50}$ values of okanin and allopurinol are shown in Table 2. The $IC_{50}$ value represents the concentration of a test material required for inhibiting 50% of xanthine oxidase activity, based on the activity of the solvent control that is designated as 100%.

Table 2

| | $IC_{50}$ (μM) |
|---|---|
| Okanin | 1.5 |
| Allopurinol | 3.1 |

As is apparent from Table 2, okanin of the present invention exhibited more potent xanthine oxidase activity than allopurinol.

(Example 4)

5-week-old male SD rats were purchased from Japan SLC, Inc. and were acclimatized for 1 week. The rats were divided into groups based on body weight (each group consisting of 6 rats), and 5 groups were designated in total; i.e., a control group, a group to which the *Artemisia* extract is administered, a group to which the chrysanthemum extract is administered, a group to which the guava extract is administered, and a group to which the oregano extract is administered.

At the outset, potassium oxonate (Sigma-Aldrich) was administered intraperitoneally to rats at a dose of 250 mg/kg of body weight to prepare potassium oxonate-induced hyperuricemic rat models. The *Artemisia* extract, the chrysanthemum extract, the guava extract, and the oregano extract obtained in Example 1 were each suspended in medium chain triglyceride, and the suspensions were administered to the group to which the *Artemisia* extract is administered, the group to which the chrysanthemum extract is administered, the group to which the guava extract is administered, and the group to which the oregano extract is administered at a dose of 500 mg of the extract per kg of body weight 1 hour after administration of potassium oxonate. Medium chain triglyceride was administered to the control group. Subsequently, blood was sampled from all rats 2 hours after administration of potassium oxonate. Blood serum was fractionated from the sampled blood via centrifugation and the uric acid level in the blood was measured. The results are shown in Table 3.

Table 3

| | Uric acid level in blood (mg/dL) |
|---|---|
| Control group | 5.80 ± 0.22 |
| Group to which *Artemisia* extract is administered | 4.42 ± 0.52 |
| Group to which chrysanthemum extract is administered | 4.70 ± 0.48 |
| Group to which guava extract is administered | 4.91 ± 0.27 |
| Group to which oregano extract is administered | 4.88 ± 0.41 |

As is apparent from Table 3, administration of the *Artemisia* extract, the chrysanthemum extract, the guava extract, and the oregano extract results in significantly lowered uric acid levels.

(Example 5)

5-week-old male SD rats were purchased from Japan SLC, Inc. and were acclimatized for 1 week. The rats were divided into groups based on body weight (each group consisting of 6 rats), and 3 groups were designated in total; i.e., a control group, a group to which the *Saussurea involucrata* extract is administered, and a group to which the cudweed *(Gnaphalium affine*) extract is administered.

At the outset, potassium oxonate (Sigma-Aldrich) was administered intraperitoneally to rats at a dose of 250 mg/kg of body weight to prepare potassium oxonate-induced hyperuricemic rat models. The *Saussurea involucrata* extract and the cudweed *(Gnaphalium affine*) extract obtained in Example 1 were each suspended in medium chain triglyceride, and the suspensions were administered to the group to which the *Saussurea involucrata* extract is administered and the group to which the cudweed *(Gnaphalium affine*) extract is administered at a dose of 1,000 mg of the extract per kg of the body weight 1 hour after administration of potassium oxonate. Medium chain triglyceride was administered to the control group. Subsequently, blood was sampled from all rats 3 hours after administration of potassium oxonate. Blood serum was fractionated from the sampled blood via centrifugation and the uric acid level in the blood was measured. The results are shown in Table 4.

Table 4

| | Uric acid level in blood (mg/dL) |
|---|---|
| Control group | 5.17 ± 0.87 |
| Group to which *Saussurea involucrata* extract is administered | 4.13 ± 1.10 |
| Group to which cudweed (*Gnaphalium affine*) extract is administered | 3.98 ± 0.97 |

As is apparent from Table 4, administration of the *Saussurea involucrata* extract and the cudweed *(Gnaphalium affine*) extract results in significantly lowered uric acid levels.

(Example 6)

5-week-old male SD rats were purchased from Japan SLC, Inc. and were acclimatized for 1 week. The rats were divided into groups based on body weight (each group consisting of 6 rats), and 2 groups were designated in total; i.e., a control group and a group to which okanin is administered.

At the outset, potassium oxonate (Sigma-Aldrich) was administered intraperitoneally to rats at a dose of 250 mg/kg of body weight to prepare potassium oxonate-induced hyperuricemic rat models. Okanin was suspended in distilled water and the resultant was administered at a dose of 50 mg of okanin per kg of the body weight 1 hour after administration of potassium oxonate. Distilled water was administered to the control group. Subsequently, blood was sampled from all rats 2 hours after administration of potassium oxonate. Blood serum was fractionated from the sampled blood via centrifugation and the uric acid level in the blood was measured. The results are shown in Table 5.

Table 5

| | Uric acid level in blood (mg/dL) |
|---|---|
| Control group | 4.92 ± 0.53 |

(continued)

| | Uric acid level in blood (mg/dL) |
|---|---|
| Group to which okanin is administered | 4.10 ± 0.56 |

As is apparent from Table 5, administration of okanin results in a significantly lowered uric acid level.

(Example 7)

*Artemisia* (whole plant), chrysanthemum (flowers), guava (leaves), cudweed (*Gnaphalium affine*) (whole plant), blue mallow (flowers), oregano (leaves and flowers), and mint (leaves) (1,000 g each) were soaked in aqueous solutions of 90%, 60%, and 30% ethanol by volume or in water, agitation and extraction were carried out at 45°C for 6 hours, and residues were removed by filtration to obtain extracted solutions. Further, the extracted solutions were concentrated under reduced pressure to remove solvents, and extracts were obtained.

(Example 8)

The extracts obtained in Example 7 were dissolved in DMSO to a concentration of 100 mg/ml. The solutions of samples in DMSO were dissolved in 75 mM phosphate buffer (pH 7.5) to prepare solutions containing samples at concentrations of 400 μg/ml.

A sample solution (50 μl) and an enzyme solution comprising buttermilk-derived xanthine oxidase dissolved at 0.2 units/ml in phosphate buffer (50 μl) were added to a 96-well plate and treated at 25°C for 15 minutes. Thereafter, 100 μl of 0.4 mM xanthine solution was added and the reaction was allowed to proceed at 25°C for an additional 15 minutes. In this case, the final concentration of the sample was 100 μg/ml. The reaction was terminated with the addition of 20 μl of 1N hydrochloric acid. The absorbance at 295 nm was assayed using a microplate reader. As a solvent control, 50 μl of a solution comprising DMSO dissolved at 0.4% in phosphate buffer was used instead of the sample solution. The amount of uric acid (i.e., the reaction value) generated by xanthine oxidase in the reaction solution comprising the solvent control or the sample was determined by subtracting the absorbance assayed in the absence of xanthine oxidase from the absorbance assayed in the presence thereof. The xanthine oxidase inhibitory activity was determined by the following formula:

$$\text{Xanthine oxidase inhibitory activity (\%)} = \{1 - (\text{reaction value of test substance (sample)} / \text{reaction value of solvent control})\} \times 100$$

Xanthine oxidase inhibitory activities of the extracts of *Artemisia*, chrysanthemum, guava, cudweed (*Gnaphalium affine*), blue mallow, oregano, and mint obtained in Example 7 are shown in Table 6.

Table 6

| | Ethanol concentration in extracted solution (vol. %) | Xanthine oxidase inhibitory activity (%) |
|---|---|---|
| *Artemisia* extract | 60 | 46.6 |
| Chrysanthemum extract | 60 | 51.7 |
| | 30 | 49.1 |
| Guava extract | 60 | 48.6 |
| | 30 | 58.4 |
| | 0 | 46.8 |
| Cudweed (*Gnaphalium affine*) extract | 60 | 77.1 |
| | 30 | 58.3 |
| Blue mallow extract | 60 | 51.9 |
| | 30 | 46.7 |

(continued)

| | Ethanol concentration in extracted solution (vol. %) | Xanthine oxidase inhibitory activity (%) |
|---|---|---|
| Oregano extract | 60 | 79.7 |
| | 30 | 57.9 |
| Mint extract | 60 | 42.6 |

As is apparent from Table 6, the extracts of *Artemisia*, chrysanthemum, guava, cudweed (*Gnaphalium affine*), blue mallow, and oregano obtained in Example 7 exhibit potent xanthine oxidase inhibitory activities.

## Claims

1. An agent for preventing or ameliorating hyperuricemia comprising, as an active ingredient, an extract of at least one type of plant raw material selected from the group consisting of *Artemisia, Saussurea involucrate*, chrysanthemum, guava, cudweed (*Gnaphalium affine*), blue mallow, oregano, *Glechoma hederacea,* mint, *Millettia reticulata*, and peanuts obtained with the use of an organic solvent or aqueous organic solvent, Pycnogenol, and/or a chalcone derivative represented by general formula (1):

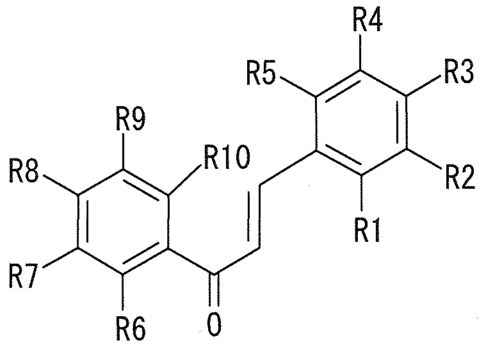

(1)

wherein R1 to R10 are each independently H, OH, or $OCH_3$, or a glycoside thereof.

2. A xanthine oxidase inhibitory agent comprising, as an active ingredient, an extract of at least one type of plant raw material selected from the group consisting of *Artemisia*, *Saussurea involucrate*, chrysanthemum, guava, cudweed *(Gnaphalium affine*), blue mallow, oregano, *Glechoma hederacea*, mint, *Millettia reticulata*, and peanuts obtained with the use of an organic solvent or aqueous organic solvent, Pycnogenol, and/or a chalcone derivative represented by general formula (1):

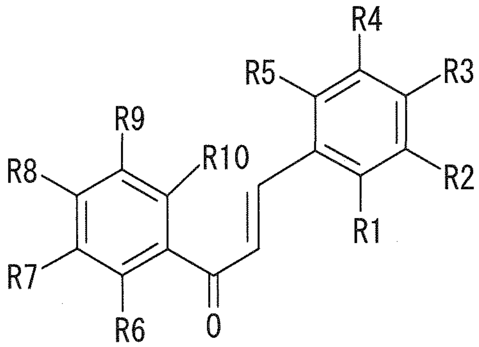

(1)

wherein R1 to R10 are each independently H, OH, or $OCH_3$, or a glycoside thereof.

3. The agent according to claim 1 or 2, wherein the organic solvent is ethanol.

4. The agent according to claim 1 or 2, wherein the chalcone derivative represented by general formula (1) or the glycoside thereof is derived from a plant of the genus *Bidens.*

5. The agent according to claim 1 or 2, wherein the chalcone derivative represented by general formula (1) or the glycoside thereof is okanin or an okanin glycoside.

6. A food or beverage product comprising the agent according to any one of claims 1 to 5.

7. The food or beverage product according to claim 6, which is a functional food.

8. The food or beverage product according to claim 6, which is a food for specified health use.

9. A cosmetic or pharmaceutical product comprising the agent according to any one of claims 1 to 5.

**INTERNATIONAL SEARCH REPORT**

International application No.
PCT/JP2009/050797

A. CLASSIFICATION OF SUBJECT MATTER
*A61K36/28*(2006.01)i, *A23L1/30*(2006.01)i, *A61K8/97*(2006.01)i, *A61K36/00*(2006.01)i, *A61P19/06*(2006.01)i, *A61P43/00*(2006.01)i, *C12N9/99*(2006.01)i

According to International Patent Classification (IPC) or to both national classification and IPC

B. FIELDS SEARCHED

Minimum documentation searched (classification system followed by classification symbols)
A61K36/28, A23L1/30, A61K8/97, A61K36/00, A61P19/06, A61P43/00, C12N9/99

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/BIOSIS/MEDLINE/WPIDS(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | JP 61-40763 A (Kabushiki Kaisha Osaka Yakuhin Kenkyusho), 27 February, 1986 (27.02.86), Test example 2 (Family: none) | 1-9 |
| X | Yun, Kyeong Won et al, The influence of the growth season on the antimicrobial and antioxidative activity in Artemisia princeps var. orientalis, Industrial Crops and Products, 2008.01, Vol.27,No.1, pp.69-74, ScienceDirect, Available[online](2007.09.05) | 1-9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 April, 2009 (07.04.09) | 21 April, 2009 (21.04.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/050797

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The following published documents 1 and 2 disclose that artemisia extracts, which are described first in claims 1 and 2, lower uric acid level and have a xanthine oxidase inhibitory effect. Accordingly, the technical features of claims 1 to 9 reside in finding an effect of lowering uric acid or inhibiting xanthine oxidase respectively in extracts of the 11 crude drugs as set forth in claims 1 and 2, picnogenol and a chalcone derivative. Thus, it is recognized that claims 1 to 9 have 13 inventions corresponding respectively to the extracts of the individual crude drugs, picnogenol and a chalcone derivative which cannot be considered as a group of inventions being so linked as to form a single general inventive concept. (Continued to extra sheet.)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Parts of claims 1 to 9 relating to artemisia.

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.
PCT/JP2009/050797

Continuation of Box No.III of continuation of first sheet(2)

Document 1: JP 61-40763 A (Kabushiki Kaisha Osaka Yakuhin Kenkyusho), 27 February, 1986 (27.02.86)
Document 2: Industrial Crops and Products, 2008.01, Vol.27, No. 1, pp.69-74

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006036787 A
- JP 2007045784 A


### Non-patent literature cited in the description


- *Purine-pyrimidine taisha (metabolism),* 1994, vol. 18, 1-10
- *Biol. Pharm. Bull.,* 2004, vol. 27, 1414-1421